# EUROPEAN PATENT SPECIFICATION

(11) **EP 1 955 702 B1**
(45) Date of publication and mention of the grant of the patent: **27.06.2012**
(21) Application number: 07117485.8
(22) Date of filing: 28.09.2007
(51) Int. Cl.: A61K 35/74, A61P 1/02, A23L 1/30, C12N 1/20

(54) **Lactobacillus paracasei-containing product for use in inhibiting dental diseases**
Lactobacillus paracasei-haltige Produkt zur Verwendung in der Hemmung von Zahnerkrankungen
Produit à base de Lactobacillus paracasei pour l'utilisation dans l'inhibition de maladies dentaires

(30) Priority: 28.11.2006 CN 200610145932
(43) Date of publication of application: 13.08.2008
(73) Proprietor: GenMont Biotech Inc., Shanhua Township T'ai nan (TW)
(72) Inventor: Hsu, Ching-Hsiang, Tainan County (TW); Chen, Ya-Hui, Tainan County (TW); Wang, Ying-Yu, Tainan County (TW); Lai, Ding-Ying, Tainan County (TW); Hsieh, Feng-Ching, Tainan County (TW)
(74) Representative: Viering, Jentschura & Partner

(56) References cited:
- WO-A-2006/048457
- WO-A-2006/088923
- PANGSOMBOON ET AL: "Antibacterial activity of a bacteriocin from Lactobacillus paracasei HL32 against Porphyromonas gingivalis" ARCHIVES OF ORAL BIOLOGY, PERGAMON PRESS, OXFORD, GB, vol. 51, no. 9, 1 September 2006 (2006-09-01), pages 784-793, XP005609312 ISSN: 0003-9969
- SOOKKHEE S ET AL: "Lactic acid bacteria from healthy oral cavity of Thai volunteers: Inhibition of oral pathogens" JOURNAL OF APPLIED MICROBIOLOGY, OXFORD, GB, vol. 90, no. 2, 1 February 2001 (2001-02-01), pages 172-179, XP002321894 ISSN: 1364-5072

## Description

### BACKGROUND OF THE INVENTION

### Field of Invention

The present invention relates to a product used in oral cavity, and more particularly, to a Lactobacillus paracasei-containing product used for inhibiting dental diseases caused by bacteria.

### Related Art

A dental caries is one of multi-factorial diseases caused by the following factors, including constituted element of teeth, configuration, position, component of saliva, pH value, secretion, viscosity, anti-bacterial factors, carbohydrate level in diet, fluoride, and microorganisms and their physical properties. Therefore, reducing the number of bacteria in oral cavity may reduce the occurrence of periodontal diseases caused by dental caries and bacteria. Generally, the bacteria affecting the health care of teeth are all in the form of a dental plaque, which is a film-shaped substance adhered on a surface of teeth in the oral cavity, with the appearance being gradually changed from a transparent appearance into a white deposit. The bacteria in the dental plaque include a variety of cocci, bacilli, and leptothrix, wherein the main bacteria of dental plaques that most easily causes dental caries or a periodontal inflammation include Streptococcus mutants, Streptococcus sanguis, Actinomyces viscosus, Streptococcus sobrinus, and Porphyromonas gingivalis.

Currently, many researching results show that, special probiotics can reduce the occurrence of dental caries, and also decrease the number of the Streptococcus mutants of dental caries in oral cavity, which further reduces the formation of dental plaques so as to decrease a seedbed for pathogens of periodontal disease, and thereby preventing gingivitis and bone loss of an inferior alveolar bone caused by the toxin of bacteria, and reducing the occurrence of periodontal diseases. For example, a study in Japan has found that a Lactobacillus salivarius (LS1) may be used to effectively prevent dental caries. Additionally, in Helsinki University of Holland, children at the age of 1-6 who have administered milk added with lactobacilli in a long term for preventing dental caries are studied; and, young people who have administered cheeses added with a special probiotic in a short term for preventing dental caries are also studied. The result shows that the number of Streptococcus mutants in saliva is reduced by approximately 20% after 3 weeks. Furthermore, a study in Mexico also shows that continually chewing a chewing gum containing lactobacilli treated by heat sterilization once a week for 16 weeks may improve dental caries and reduce the occurrence of dental caries by approximately 42%.

Additionally, several kinds of lactobacillus cultures for inhibiting the formation of the dental plaque are disclosed in U.S. Patent No. 6,036,952, which include Enterococcus spp. 1357, Lactobacillus acidophilus V20, and Lactococcus lactis 1370. In addition, a product containing lactobacillus cultures of ATTC PTA-4965 and PTA-4964 is disclosed in U.S. Patent No. 6,872,565, which is used for inhibiting dental caries caused by bacteria. The inhibition effect achieved by combining the ATTC PTA-4965 or PTA-4964 and an oral mucin or the dental plaque is used for effectively reducing the number of Streptococcus mutants. Alternatively, treating and preventing dental caries, dental plaques, and periodontal diseases with lactobacillus cultures CNCM I-1984, CNCM I-1985, CNCM I-1986, CNCM I-1987, or LMG P-18997 is disclosed in U.S. Patent No. 6,942,849, wherein the lactobacillus cultures are genetically modified to enhance the viscosity for a tooth surface film.

Currently, it is found that, Lactobacillus paracasei may be used to promote an immunocyte to release a number of interferon, which is helpful for improving allergic diseases. Human immunocyte T lymphocytes are divided into two types according to the secreted different cytokines, wherein there is a dynamic balance between Type I and Type II T lymphocytes, and both of them are influenced by each other, thereby affecting the result of a reaction of the whole immune system to an antigen. If the reaction of Type II T lymphocyte is excessively strong, an allergic symptom occurs to a patient. The allergy may be prevented by increasing the activity of Type I T lymphocyte or inhibiting the activity of Type II T lymphocyte. When the Lactobacillus paracasei is used in human bowel, due to the difference of cell walls and cell contents, the Lactobacillus paracasei significantly promotes the activity of Type I T lymphocyte and further inhibits the effect of Type II T lymphocyte, and thus preventing allergy.

To sum up, a variety of lactobacilli are now found to have the function of inhibiting dental caries, dental plaques, and periodontal diseases. The Lactobacillus paracasei has been used in human bowel in the past to relieve the body allergic symptom after being absorbed by the bowel. However, the efficacy that the Lactobacillus paracasei is used in oral cavity to prevent dental diseases has not been found yet.

### SUMMARY OF THE INVENTION

The prevent invention provides a Lactobacillus paracasei-containing product, which is used for inhibiting dental diseases caused by bacteria, such as dental caries, dental plaques, and periodontal diseases.

The Lactobacillus paracasei-containing product disclosed in the prevent invention is used for inhibiting dental diseases caused by bacteria, which comprises a plurality of Lactobacillus paracasei for inhibiting the growth of bacteria of dental diseases, wherein the deposit designation of the culture for the Lactobacillus paracasei is CCTCC is 206133.

Foods, oral hygiene products or oral treatment medicine containing the Lactobacillus paracasei when being administered or applied to a user can inhibit or reduce the number of pathogens of dental caries and periodontal diseases in oral cavity of the user, thereby achieving the efficacy of preventing dental diseases, such as dental caries and periodontal diseases.

Further scope of applicability of the present invention will become apparent from the detailed description given hereinafter.

### BRIEF DESCRIPTION OF THE DRAWINGS

The present invention will become more fully understood from the detailed description given herein below for illustration only, and thus is not limitative of the present invention, and wherein:
FIG. 1 is a DNA finger map of a Lactobacillus paracasei;
FIGs. 2A and 2B are results of an antimicrobial cycle test for inhibiting the pathogens of dental caries with the Lactobacillus paracasei;
FIG. 3A is a result of an animal test in a control group;
FIG. 3B is a result of an animal test after inoculation of a pathogen S. sobrinus;
FIG. 3C is a result of an animal test for inhibiting the pathogen with a sodium fluoride after inoculation of the pathogen S. sobrinus;
FIG. 3D is a result of an animal test for inhibiting the pathogen with the Lactobacillus paracasei at a low bacteria count after inoculation of the pathogen S. sobrinus;
FIG. 3E is a result of an animal test for inhibiting the pathogen with the Lactobacillus paracasei at a high bacteria count after inoculation of the pathogen S. sobrinus;
FIG. 4A shows a result for inhibiting the growth of pathogens of periodontal disease with an active Lactobacillus paracasei; and
FIG. 4B shows a result for inhibiting the growth of pathogens of periodontal disease with a sterilized Lactobacillus paracasei.

### DETAILED DESCRIPTION OF THE INVENTION

To further understand the objective, structure, feature, and function of the present invention, the present invention is illustrated below in great detail through the embodiments. The above illustration about the summary of the prevent invention and the following illustration about the detailed description of the present invention are intended to demonstrate and explain the principle of the present invention, and provide further explanations for claims of the prevent invention.

### I. Screening and Identification of the Lactobacillus Paracasei:

First of all, a functional lactobacillus culture for alleviating dental disease symptoms is screened from the lactobacillus cultures existed in human bowel, which is a special lactobacillus culture for preventing dental caries screened from lactobacillus cultures in a culture collection center via the antimicrobial cycle test with the main pathogens Streptococcus mutans and Streptococcus sobrinus that cause the formation of dental caries. This functional lactobacillus culture is incubated in a culture dish without oxygen at a culture temperature of 37°C for 16-24 hours. It is found in an experimental analysis that this lactobacillus culture is a Gram-positive bacteria, a non-sporing anaerobes, and a non-mobility, which is consistent with the property of the Lactobacillus paracasei.

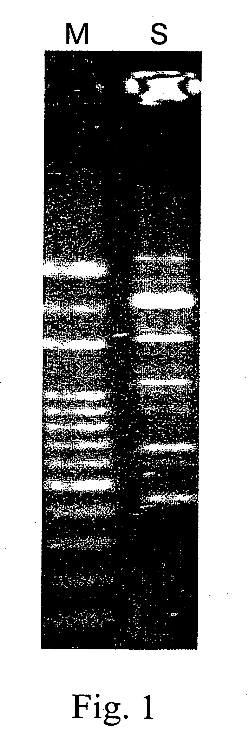

To confirm whether the screened lactobacillus culture is the Lactobacillus paracasei, the assay of random amplified polymorphic DNA-polymerase chain reaction (RAPD-PCR) is carried out for the lactobacillus culture. As shown in FIG. 1, the RAPD-PCR technology is used to generate a DNA fingerprint S of the lactobacillus culture with an arbitrary primer. After comparison, it is found that the DNA fingerprint S does consistent with the Lactobacillus paracasei and it is different with other lactobacillus cultures. M in the figure indicates a tagged molecule of molecular weight.

Then, a nucleotide sequencing is carried out for the lactobacillus culture with PAF and 536R primers. The sequencing result is compared with a gene database (the website is www.ncbi.nlm.nih.gov), and then, it is confirmed that this lactobacillus culture is the Lactobacillus paracasei. A part of the nucleotide sequencing result of the lactobacillus culture is shown as follows:

AACGAGTTCTCGTTGATGATCGGTGCTTGCACCGAGATTCAACATGGAA
CGAGTGGCGGACGGGTGAGTAACACGTGGGTAACCTGCCCTTAAGTGGGGG
ATAACATTTGGAAACAGATGCTAATACCGCATAGATCCAAGAACCGCATGGTT
CTTGGCTGAAAGATGGCGTAAGCTATCGCTTTTGGATGGACCCGCGGCGTATT
AGCTAGTTGGTGAGGTAATGGCTCACCAAGGCGATGATACGTAGCCGAACTG
AGAGGTTGATCGGCCACATTGGGACTGAGACACGGCCCAAACTCCTACGGG
AGGCAGCAGTAGGGAATCTTCCACAATGGACGCAAGTCTGATGGAGCAACG
CCGCGTGAGTGAAGAAGGCTTTCGGGTCGTAAAACTCTGTTGTTGGAGAAG
AATGGTCGGCAGAGTAACTGTTGTCGGCGTGACGGTATCCAACCAGAAAGCC

ACGGCTAACTACGTGCCAGCAGCGNGGGTTAATTAAAA

The comparison result of the gene database is shown as follows:
gi|55418407|gb|AY773955.1| Lactobacillus paracasei isolate 9C...
gi|55418405|gb|AY773953.1| Lactobacillus paracasei isolate 3C...
gi|55418404|gb|AY773952.1| Lactobacillus paracasei isolate 2C.......
Finally, the carbohydrate metabolism performance of the lactobacillus culture is tested with an API 50 CHL reagent. The API 50 CHL reagent is used for identifying the difference of cultures in genus or species. It is determined by analyzing the result that, the activity of carbohydrate metabolism of the lactobacillus culture is the same as that of the Lactobacillus paracasei (not shown in data).

The screened special lactobacillus culture for preventing dental diseases is determined to be the Lactobacillus paracasei after being identified by the above analysis result.

### II. Inhibition Effect of the Lactobacillus Paracasei for the Pathogen of Dental Caries:

### 1. Bacteria Test

An antimicrobial cycle test for inhibiting the pathogen of dental caries is carried out after being modified according to the method of the National Committee for Clinical Laboratory Standards (NCCLS). A platinum loop of pathogens of dental caries is inoculated in a desired culture medium. The strain is incubated at 37°C for 18 hours to reach a log-growth phase. The bacteria count is measured with a Smart Spec™ 3000 Spectrophtometer, and the count of pathogens of dental caries is adjusted to 1.5× 10⁸ CFU/mL. The Streptococcus mutans and Streptococcus sobrinus are uniformly applied on the desired culture medium with a thickness of 4 mm, which is stand for 15 minutes, such that the coated bacteria solution is completely adhered on the culture medium. An asepsis filter paper disc with a diameter of 6 mm is used to soak and receive the solution of the Lactobacillus paracasei. The asepsis filter paper disc is gently pressed to be completely adhered on the culture medium that is uniformly coated with the Streptococcus mutans and Streptococcus sobrinus. The size of the antimicrobial cycle is judged and read after incubation at 37°C for 72 hours.

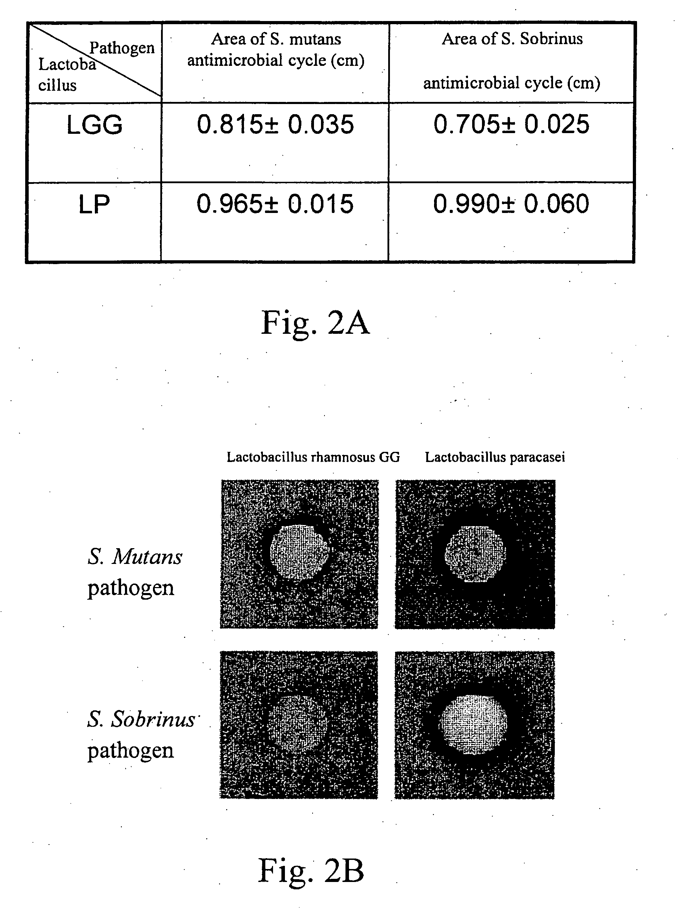

Referring to FIGs. 2A and 2B, they are results of the antimicrobial cycle test for inhibiting the pathogens of dental caries. After the Lactobacillus paracasei and the pathogens of dental caries were incubated together at 37°C fo r 72 hours, the size of the antimicrobial cycle of the main pathogens Streptococcus mutans (S. mutans) and Streptococcus sobrinus (S. sobrinus) that cause dental caries is judged and read. The result of the test shows that, the antimicrobial cycle caused by the Lactobacillus paracasei for the two trains of pathogens is larger than that of a Lactobacillus rhamnosus GG, i.e., the competitive antagonize effect of the Lactobacillus paracasei for the Streptococcus mutants and Streptococcus sobrinus is more preferable than that of the Lactobacillus rhamnosus GG. The Lactobacillus rhamnosus GG is a lactobacillus culture now widely used in healthy foods for reducing the occurrence of dental caries. Therefore, using the Lactobacillus paracasei in oral cavity is helpful for reducing the number of the Streptococcus mutants, the occurrence of dental caries, and the occurrence of the periodontal diseases caused by dental caries and bacteria.

### 2. Animal Test:

Female pregnant Sprague-Dawley (SD) mice are bought from the National Laboratory Animal Center. When infant mice are 18 days old, a 0.2 ml culture solution incubated overnight for the pathogen S. sobrinus of dental caries at a log-growth phase is touched with a cotton stick, and then, the culture solution is applied in the oral cavity of female mice for inoculability. A 10% aqueous sucrose solution is available for the female mice without limitation. The step of inocuability is repeated at the second day. The caries bacteria S. sobrinus is applied in the oral cavity of the female mice with a sterile cotton stick at the third day of inocuability; and then, it is incubated in a Mitis Salivarius culture medium containing 100 µg/ml Streptomycin; finally, it is inspected whether the inocuability is successful.

The SD infant mice are ablactated on the 20^{th} day, and then, they are divided into groups. A 0.2 ml culture solution incubated overnight for the pathogen S. sobrinus of dental caries at a log-growth phase is touched with a cotton stick, and then, the culture solution is applied in the oral cavity of SD infant mice for inoculability. A 10% aqueous sucrose solution is available for the SD infant mice without limitation. The step of inocuability is repeated at the second day. The caries bacteria S. sobrinus is applied in the oral cavity of the SD infant mice with a sterile cotton stick at the third day of inocuability; and then, it is incubated in a Mitis Salivarius culture medium containing 100 µg/ml Streptomycin; finally, it is inspected whether the inocuability is successful.

The aqueous sucrose solution is available for the SD infant mice without limitation during the period from the 23^{rd} day since the mice were born to the completion of the test. Body weights are measured before the efficacy test, and the body weights are measured once a week during the test. The SD infant mice are sacrificed after 5 weeks for observing the degree of dental caries.

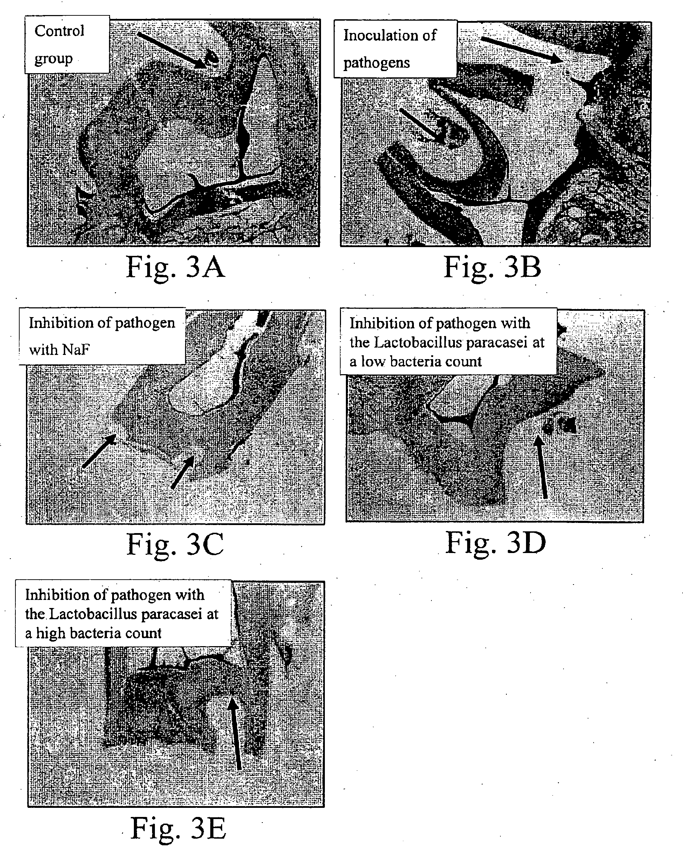

Referring to FIGs. 3A-3E, they show results of the animal test for inhibiting the pathogens of dental caries. FIG. 3A is a test result of a bank group. The pathogens of dental caries are not incubated, and the aqueous sucrose solution added with the Lactobacillus paracasei is not available throughout the test. FIG. 3B is a test result of the inoculation of a pathogen S. sobrinu. FIG. 3C is a test result for using an aqueous sucrose solution containing 0.022% NaF after inoculation of the pathogen S. sobrinus. FIG. 3D is a test result for using an aqueous sucrose solution containing 5 × 10⁹ CFU/ml Lactobacillus paracasei after inoculation of the pathogen S. sobrinus. FIG. 3E is a test result for using an aqueous sucrose solution containing 5 × 10¹¹ CFU/ml Lactobacillus paracasei after inoculation of the pathogen S. sobrinus.

The test conditions in FIG. 3C include that the aqueous sucrose solution containing 0.022% NaF is available for the mice from the 23^{rd} day after the inoculation for the pathogen S. sobrinus of dental caries is successful; the pH value of the aqueous solution is 7; the concentration of Fluoride ion is 100 ppm. The test conditions in FIGs. 3D and 3E include that the Lactobacillus paracasei at the bacteria count of 5 × 10⁹ CFU/ml and 5 × 10¹¹ CFU/ml are respectively fed to the mice at 23 days after the inoculation for the pathogen S. sobrinus of dental caries is successful. Each SD mouse is fed with 0.05 ml on left and right cheek respectively once a day, which is totally fed with 0.1 ml of the Lactobacillus paracasei each day.

The test for the above test groups lasts for 5 weeks. The tooth of the mice is sliced to observe the depth caused by dental caries after the test is completed. The result of FIG. 3B indicates that, after the inoculation of the S. sobrinus culture solution, the aqueous sucrose solution is available without limitation for 5 weeks, so that the depth of dental caries is the most serious even to invade the part of the tooth marrow as indicated by the black arrows in FIG. 3B. But only low dental caries were formed, as indicated by the black arrows, when adding NaF (as shown in FIG. 3C) or the Lactobacillus paracasei (as shown in FIGs. 3D and 3E) to the aqueous sucrose solution, and the depth of the low dental caries is similar to that caused by dental caries in the control group (as shown in FIG. 3A). This test shows that, the efficacies in preventing the formation of dental caries are equivalent when administering an aqueous sucrose solution containing the Lactobacillus paracasei or added with NaF.

### III. Inhibition Effect of the Lactobacillus Paracasei for the Pathogens of Periodontal Disease:

The pathogens of periodontal disease selected in this test include Porphyromonas gingivalis, Prevotella intermedia, Centipeda periodontii, and a clinic sample of the periodontal disease bacteria obtained from the cheek tooth of a patient on periodontal disease. The pathogens of periodontal disease and the Lactobacillus paracasei are shaking-incubated together. After being inoculated for 0, 1, 2, 4, 6, and 12 hours, 5 µl culture solution is taken from the culture solution with 10-fold and 100-fold serial dilution, and then applied on the BBAP culture medium. They are placed in an anaerobic incubator at 37°C for standing and incubating for 48-96 hours. After colonies have been formed, a colony counter is used to calculate the obtained colonies. It is determined whether there is an inhibition effect of the active Lactobacillus paracasei and the sterilized Lactobacillus paracasei (dead Lactobacillus paracasei) for the pathogens of periodontal disease.

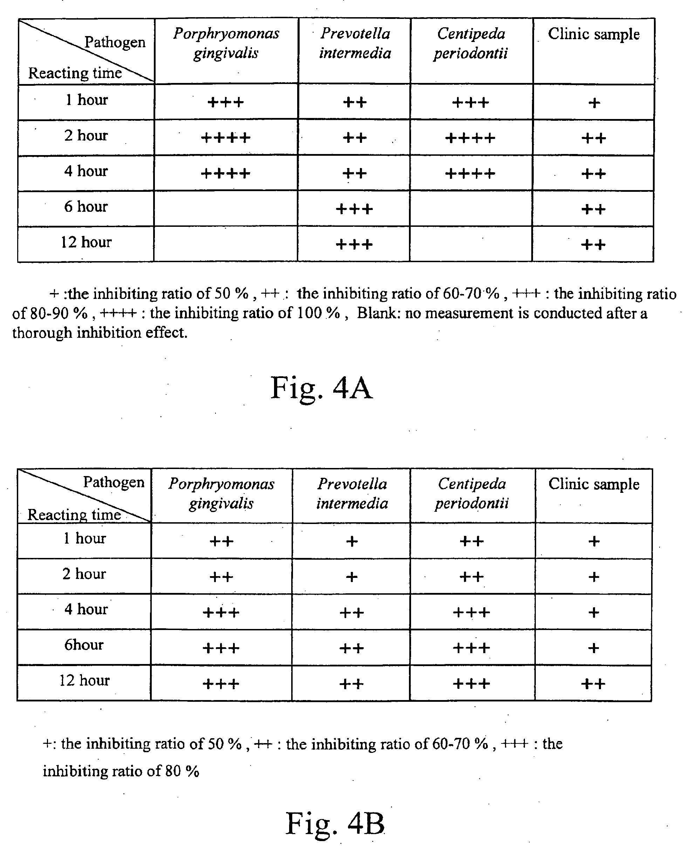

Referring to FIG. 4A, it shows a result for inhibiting the growth of pathogens of periodontal disease with an active Lactobacillus paracasei. As shown in the table that, the active Lactobacillus paracasei indeed causes the phenomenon of inhibiting the growth of pathogens of periodontal disease, and when the pathogens Porphyromonas gingivalis and Centipeda periodontii are respectively combined and reacted with the active Lactobacillus paracasei for 1 hour, over 80% inhibiting ratio is achieved, and when the above two pathogens are reacted with the active Lactobacillus paracasei for 2 hours, the two pathogens (an inhibiting ratio of 100% ) will be completed eliminated. As for the inhibition effect for Prevotella intermedia, when it is combined and reacted with the active Lactobacillus paracasei for 1 hour, an inhibiting ratio of about 70% is achieved, and a maximum inhibiting ratio (an inhibiting ratio of 80-90%) occurs after it is combined and reacted with the active Lactobacillus paracasi for 6 hours. The only tested clinic sample is the specimen of the dental plaques under the gingiva. Since the clinic sample is a combined flora, the symbiosis may enhance the pathogens' resistance, and an inhibiting ratio of about 50% is achieved after being combined and reacted with the active Lactobacillus paracasei for 1 hour, and the inhibiting ratio is increased to about 70% after 2 hours, and the inhibiting ratio is changed slightly as the prolonging of the combining and reacting duration.

Referring to FIG. 4B, it shows a result for inhibiting the growth of pathogens of periodontal disease with a sterilized Lactobacillus paracasei. The inhibition effect of the essentially sterilized Lactobacillus paracasei for the growth of pathogens of periodontal disease is lower than that of the active Lactobacillus paracasei. As for the pathogens of periodontal disease, an inhibiting ratio of 50-70% is achieved after combining and reacting the dead Lactobacillus paracasei with the pathogens of periodontal disease for 1 hour. As for the pathogens Porphyromonas gingivalis and Centipeda periodontii of periodontal disease, an inhibiting ratio close to 80% is achieved after combining and reacting the dead Lactobacillus paracasei with the pathogens Porphyromonas gingivalis and Centipeda periodontii of periodontal disease for 4 hours. As for the Prevotella intermedia, an inhibiting ratio close to 60% is achieved after combining and reacting the dead Lactobacillus paracasei with the Prevotella intermedia for 4 hours. As for the clinic sample, an inhibiting ratio of less than 50% is achieved after combining and reacting the clinic sample with the Prevotella intermedia for 1 hour. The maximum inhibiting ratio occurs after the combining process has lasted for 12 hours (inhibiting ratio of about 60%).

The result of this test shows that, although the inhibition effect of the sterilized Lactobacillus paracasei for pathogens of periodontal disease is weaker than that of the active Lactobacillus paracasei, the sterilized Lactobacillus paracasei still has the inhibition effect.

### IV Conclusion:

The high occurrence of dental caries is determined in clinic according to whether the bacteria count of the pathogen Streptococcus mutans is higher than 10⁶ CFU/ml. Therefore, the special lactobacillus strain for preventing dental caries is screened by the antimicrobial cycle test of the lactobacillus cultures for the pathogens Streptococcus mutans and Streptococcus sobrinus of dental caries, which is aimed at inhibiting the growth of the Streptococcus mutans by using the lactobacilli, and thereby reducing the occurrence of dental caries. It is confirmed via a series of inhibition tests that, the Lactobacillus paracasei has the efficacy of inhibiting dental caries and pathogens of periodontal disease.

The widely known health care function of the Lactobacillus paracasei lies in enhancing immunity and preventing allergy. However, when the products containing the Lactobaeillus paracasei produced according to the prevent invention, such as food, oral care products or oral treatment medicine, are administered or used by a user, no matter the products contain the active Lactobacillus paracasei or the dead Lactobacillus paracasei, they inhibit the pathogens of dental caries and periodontal disease in oral cavity of the user with the Lactobacillus paracasei, so as to achieve the efficacy of treating and preventing dental caries and periodontal disease. The functional lactobacillus Lactobacillus paracasei suitable for human body is used for preventing the dental caries and alleviating the periodontal disease symptoms. When the products containing the Lactobacillus paracasei are used in oral cavity for treating dental diseases, they do not cause the non-adaptation of human body, and also do not have the risk as typical noxious fluorides for curing the dental caries.

A deposit designation of a culture of the Lactobacillus paracasei in the prevent invention is CCTCC M 206133. The food containing the Lactobacillus paracasei of the prevent invention may be a liquid food, for example, drink products added with the Lactobacillus paracasei such as juice, milk, and tea. The food may also be a solid food such as confection, troche, biscuit, chocolate, and cheese added with the Lactobacillus paracasei. In addition, the food may also a gel food, such as yoghourt, jelly, pudding; and gum added with the Lactobacillus paracasei. This type of food is suitable for children to prevent dental caries. The product containing the Lactobacillus paracasei in the prevent invention also includes an oral hygiene product, such as toothpastes and agargle added with the Lactobacillus paracasei, and the Lactobacillus paracasei is used to replace the typical fluoride for inhibiting the pathogens of dental disease and thereby provide a safe and effective protection for teeth. In addition, the product containing the Lactobacillus paracasei also includes an oral treatment medicine, such as ointments and troches added with the Lactobacillus paracasei. The ointments are applied on the surface of teeth or the troches are used in oral cavity in a long term, such that the time duration for the Lactobacillus paracasei to be reacted with the pathogens is prolonged, thus, the growth of the pathogens is more effectively inhibited.

## Claims

1. A Lactobacillus paracasei-containing product for use in inhibiting dental diseases caused by bacteria, comprising a plurality of Lactobacillus paracasei used for inhibiting the growth of bacteria of dental diseases, wherein the deposit designation of the culture for the Lactobacillus paracasei is CCTCC M 206133.

2. The Lactobacillus paracasei-containing product for use as claimed in claim 1, wherein the product is a liquid food.

3. The Lactobacillus paracasei-containing product for use as claimed in claim 2, wherein the liquid food includes juice, milk, and tea.

4. The Lactobacillus paracasei-containing product for use as claimed in claim 1, wherein the product is a solid food.

5. The Lactobacillus paracasei-containing product for use as claimed in claim 4, wherein the solid food includes confection, troche, biscuit, chocolate, and cheese.

6. The Lactobacillus paracasei-containing product for use as claimed in claim 1, wherein the product is a gel food.

7. The Lactobacillus paracasei-containing product for use as claimed in claim 6, wherein the gel food includes yoghourt, jelly, pudding, and gum.

8. The Lactobacillus paracasei-containing product for use as claimed in claim 1, wherein the product is an oral hygiene product.

9. The Lactobacillus paracasei-containing product for use as claimed in claim 8, wherein the oral hygiene product is toothpaste.

10. The Lactobacillus paracasei-containing product for use as claimed in claim 8, wherein the oral hygiene product is a gargle.

11. The Lactobacillus paracasei-containing product for use as claimed in claim 1, wherein the product is an oral treatment medicine.

12. The Lactobacillus paracasei-containing product for use as claimed in claim 11, wherein the oral treatment medicine is an ointment.

13. The Lactobacillus paracasei-containing product for use as claimed in claim 11, wherein the oral treatment medicine is a troche.

## Patentansprüche

1. Ein Produkt, das Lactobacillus paracasei enthält, zur Verwendung bei der Hemmung von Zahnkrankheiten, die durch Bakterien verursacht werden, umfassend eine Vielzahl von Lactobacillus paracasei, die bei der Hemmung des Wachstums von Bakterien von Zahnkrankheiten verwendet werden, wobei die Hinterlegungskennzeichnung der Kultur des Lactobacillus paracasei CCTCC M 206133 ist.

2. Das Produkt, das Lactobacillus paracasei enthält, zur Verwendung wie in Anspruch 1 beansprucht, wobei das Produkt ein flüssiges Lebensmittel ist.

3. Das Produkt, das Lactobacillus paracasei enthält, zur Verwendung wie in Anspruch 2 beansprucht, wobei das flüssige Lebensmittel Saft, Milch und Tee einschließt.

4. Das Produkt, das Lactobacillus paracasei enthält, zur Verwendung wie in Anspruch 1 beansprucht, wobei das Produkt ein festes Lebensmittel ist.

5. Das Produkt, das Lactobacillus paracasei enthält, zur Verwendung wie in Anspruch 4 beansprucht, wobei das feste Lebensmittel Konfekt, Pastillen, Plätzchen, Schokolade und Käse einschließt.

6. Das Produkt, das Lactobacillus paracasei enthält, zur Verwendung wie in Anspruch 1 beansprucht, wobei das Produkt ein gelförmiges Lebensmittel ist.

7. Das Produkt, das Lactobacillus paracasei enthält, zur Verwendung wie in Anspruch 6 beansprucht, wobei das gelförmige Lebensmittel Joghurt, Gelee, Pudding und Weingummi einschließt.

8. Das Produkt, das Lactobacillus paracasei enthält, zur Verwendung wie in Anspruch 1 beansprucht, wobei das Produkt ein Mundhygieneprodukt ist.

9. Das Produkt, das Lactobacillus paracasei enthält, zur Verwendung wie in Anspruch 8 beansprucht, wobei das Mundhygieneprodukt Zahnpasta ist.

10. Das Produkt, das Lactobacillus paracasei enthält, zur Verwendung wie in Anspruch 8 beansprucht, wobei das Mundhygieneprodukt eine Mundspülung ist.

11. Das Produkt, das Lactobacillus paracasei enthält, zur Verwendung wie in Anspruch 1 beansprucht, wobei das Produkt ein Medikament für eine orale Behandlung ist.

12. Das Produkt, das Lactobacillus paracasei enthält, zur Verwendung wie in Anspruch 11 beansprucht, wobei das Medikament für die orale Behandlung eine Salbe ist.

13. Das Produkt, das Lactobacillus paracasei enthält, zur Verwendung wie in Anspruch 11 beansprucht, wobei das Medikament für die orale Behandlung eine Pastille ist.

## Revendications

1. Produit contenant du Lactobacillus paracasei destiné à être utilisé pour inhiber des maladies dentaires causées par des bactéries, comprenant une pluralité de Lactobacillus paracasei utilisés pour inhiber la croissance de bactéries de maladies dentaires, la référence de dépôt de la culture de Lactobacillus paracasei étant CCTCC M 206133.

2. Produit contenant du Lactobacillus paracasei destiné à une utilisation selon la revendication 1, où le produit est un aliment liquide.

3. Produit contenant du Lactobacillus paracasei destiné à une utilisation selon la revendication 2, où l'aliment liquide comprend un jus, du lait et du thé.

4. Produit contenant du Lactobacillus paracasei destiné à une utilisation selon la revendication 1, où le produit est un aliment solide.

5. Produit contenant du Lactobacillus paracasei destiné à une utilisation selon la revendication 4, où l'aliment solide comprend un électuaire, une tablette, un biscuit, du chocolat et du fromage.

6. Produit contenant du Lactobacillus paracasei destiné à une utilisation selon la revendication 1, où le produit est un aliment sous forme de gel.

7. Produit contenant du Lactobacillus paracasei destiné à utilisation selon la revendication 6, où le produit sous forme de gel comprend un yaourt, une gelée, une crème et une gomme.

8. Produit contenant du Lactobacillus paracasei destiné à une utilisation selon la revendication 1, où le produit est un produit d'hygiène buccale.

9. Produit contenant du Lactobacillus paracasei destiné à une utilisation selon la revendication 8, où le produit d'hygiène buccale est une pâte dentifrice.

10. Produit contenant du Lactobacillus paracasei destiné à une utilisation selon la revendication 8, où le produit d'hygiène buccale est un gargarisme.

11. Produit contenant du Lactobacillus paracasei destiné à une utilisation selon la revendication 1, où le produit est un médicament pour traitement buccal.

12. Produit contenant du Lactobacillus paracasei destiné à une utilisation selon la revendication 11, où le médicament pour traitement buccal est un onguent.

13. Produit contenant du Lactobacillus paracasei destiné à une utilisation selon la revendication 11, où le médicament pour traitement buccal est une tablette.
